# EUROPÄISCHE PATENTSCHRIFT

(11) **EP 1 873 666 B1**
(45) Veröffentlichungstag und Bekanntmachung des Hinweises auf die Patenterteilung: **25.03.2009**
(21) Anmeldenummer: 06013190.1
(22) Anmeldetag: 27.06.2006
(51) Int. Cl.: G06F 19/00

(54) **Medizintechnisches Markertracking mit Bestimmung der Markereigenschaften**
Medical marker tracking system with determination of the marker characteristics
Suivi de marqueurs médicaux avec détermination des propriétés des marqueurs

(43) Veröffentlichungstag der Anmeldung: 02.01.2008
(73) Patentinhaber: BrainLAB AG, 85622 Feldkirchen (DE)
(72) Erfinder: Goldbach, Günter, 85457 Wörth/Wifling (DE)
(74) Vertreter: Schwabe - Sandmair - Marx

(56) Entgegenhaltungen:
- US-A1- 2003 174 401
- US-A1- 2003 225 329
- US-A1- 2005 049 485
- US-B1- 6 738 656

## Beschreibung

Die Erfindung betrifft das Gebiet des medizintechnischen Markertrackings, bei dem mittels eines medizintechnischen Markertrackingsystems Instrumenten- oder Patientenmarker erfasst werden. Markertrackingsysteme im allgemeinen und natürlich auch dasjenige gemäß der vorliegenden Erfindung werden in der Medizintechnik dazu benutzt, behandlungsunterstützende Geräte, Instrumente, oder Patientenkörperteile positionell zu bestimmen und zu verfolgen. Mit den Positionsdaten für die Instrumente ist es möglich, eine medizintechnische Navigation durchzuführen, die Ärzte bei der Patientenbehandlung unterstützt. Es wird dadurch möglich, eine bildgestützte Chirurgie umzusetzen, bei der Ärzte durch eine Bildschirmausgabe die Lage ihrer Instrumente gegenüber der Lage von Behandlungszielen (Patientenkörperteilen) sehen und überprüfen können, auch wenn Teile der Instrumente nicht mehr sichtbar sind. Auch die Planung von Eingriffen wird ermöglicht bzw. stark vereinfacht. Die Daten über die Patientenanatomie stammen aus vorab oder intraoperativ ermittelten Erfassungen mit bildgebenden Verfahren, z.B. Computertomographie, Kernspintomographie, Röntgenbildgebung, etc., und sie werden durch einen Registrierungsvorgang in das Koordinatensystem des Operationsraums bzw. des Instrumenten-Trackingsystems eingeordnet.

Die DE 196 39 615 beschreibt beispielsweise ein chirurgisches Navigationssystem mit Markertracking.

Die US 2003/0225329 A1 betrifft ein medizinisches Trackingsystem mit Universalsbhnittstelle.

Die US 2005/0049485 A1 betrifft ein Mehrfachkonfigurations-Array für ein chirurgisches Navigationssystem.

Die US 2003/0174401 A1 bezieht sich auf ein Reflektorsystem zum Bestimmen der Position.

Die US 6,738,656 B1 betrifft ein automatisches Registrierungssystem für die Verwendung mit Positionstracking eines Bildaufbereitungssystems zur Verwendung in medizinischen Anwendungen.

Während der Verwendung eines Trackingsystems kommt es - gerade bei Orthopädieoperationen - oftmals zur Beeinträchtigung der Markerqualität, weil die Marker teilweise oder vollständig abgedeckt werden, beispielsweise bei Verschmutzungen durch Wasser, Blut oder sonstige auftretende Flüssigkeiten oder Feststoffe.

Wenn Marker ausfallen oder in ihrer Qualität beeinträchtigt sind, verschlechtert dies die Funktionalität oder die Genauigkeit des Markertrackings. Es ist eine Aufgabe der vorliegenden Erfindung, negative Auswirkungen solcher Beeinträchtigungen der Markerqualität auf die Trackinggenauigkeit auszuschließen oder zumindest zu minimieren und die Qualität der Marker automatisiert zu überprüfen und für weitere Verwendungszwecke zu berücksichtigen.

Diese Aufgabe wird durch ein medizintechnisches Markertrackingverfahren gemäß dem Anspruch 1 sowie durch eine medizintechnische Markertrackingvorrichtung gemäß dem Anspruch 18 gelöst. Die Unteransprüche definieren bevorzugte Ausführungsformen der Erfindung.

Gemäß einem ersten Aspekt betrifft die vorliegende Erfindung ein medizintechnisches Markertrackingverfahren, bei dem mittels eines medizintechnischen Markertrackingsystems Instrumenten- oder Patientenmarker erfasst werden, wobei mittels einer Datenverarbeitungseinheit die Ist-Eigenschaften der Marker mit Soll-Eigenschaften verglichen werden und wobei das Ergebnis des Vergleichs der Weiterverwendung der Informationen über die erfassten Marker zugrunde gelegt wird.

Mit anderen Worten schlägt die vorliegende Erfindung vor, die Markerqualität während des Trackingvorganges zu überprüfen. Mit dem durch diese Überprüfung gewonnenen Informationen lässt sich feststellen, ob das Tracking noch mit der erforderlichen Genauigkeit durchgeführt werden kann, so dass die Voraussetzungen vorliegen, im Falle einer nicht mehr vorhandenen, ausreichenden Genauigkeit entsprechende Maßnahmen zu ergreifen. Dies ist von besonderem Vorteil, weil das Behandlungspersonal oftmals auf Beeinträchtigungen der Markerqualität durch Verschmutzung nicht aufmerksam wird, beispielsweise wenn der Marker nur teilweise verdeckt oder nur durch einen Wasserfilm verschmutzt wird. Die Qualität, die Sicherheit und die Zuverlässigkeit von Markertrackingsystemen werden demnach durch die vorliegende Erfindung stark verbessert.

Die oben erwähnten, zu vergleichenden Eigenschaften können die Helligkeit, die Form, die Größe, die Sichtbarkeit, den Verschmutzungsgrad und die Position (bzw. relative Position zu einer Referenz) eines Markers bzw. dessen Abbildung im Trackingsystem oder eine Kombination aus zweien oder mehreren dieser Eigenschaften umfassen. Weitere wichtige Eigenschaften können der Helligkeitsverlauf in den Sensordaten über den Marker sein und die Korrelation von Abbildungen eines Markers aus verschiedenen Blickrichtungen. Bei stereoskopischen Systemen ist eine für die dreidimensionale Positionsberechnung wichtige Eigenschaft die so genannte Sehstrahlabweichung oder auch Epipolargeometrie, die idealerweise Null sein sollte. Bei verschmutzten Markern wird sie gegenüber idealen (gut rückstrahlenden) Markern größer. Dies ergibt sich aus der Tatsache, dass die Schwerpunkte in den beiden zweidimensionalen Kamerabildern eines stereoskopischen Markertrackingsystems bei Markerverschmutzungen an unterschiedlichen Positionen berechnet werden und damit schlechter auf einen genauen Punkt zu triangulieren sind als bei guten Markern. Wenn beispielsweise ein Marker vom Kamerasystem her gesehen nur im linken Bereich eine Verschmutzung hat, dann wird die linke Kamera einen zweidimensionalen Flächenschwerpunkt berechnen, der zu weit rechts liegt. Die rechte Kamera wird davon weniger betroffen sein, weil von ihr aus die Verschmutzung unter Umständen nicht sichtbar ist. Bei der Triangulation wird nun versucht, die zum jeweiligen 2D-Flächenschwerpunkt gehörenden Sehstrahlen im Raum zu überschneiden. Dies funktioniert im Grunde nur dann bzw. nur dann gut, wenn die Schwerpunkte exakt bestimmt werden und sich die Sehstrahlen an genau einem Punkt, nämlich der Markerposition schneiden. Realiter ist das zwar nie exakt der Fall, aber bei guten Markern und einer guten Kamerakalibrierung erhält man sehr kleine, akzeptierbare Sehstrahlabweichungen. Bei schlechten Markern werden die Sehstrahlabweichungen größer und damit wird die Sehstrahlabweichung wiederum eine zu berücksichtigende Eigenschaft der Marker, weil sie von deren Eigenschafen abhängig ist. Auch wenn man die Sehstrahlabweichungen verschiedener Marker im Trackingfeld relativ zueinander betrachtet, kann man die Sehstrahlabweichung auf die Qualität einzelner Marker zurückführen.

Um einen speziellen Marker über eine Zeitspanne verfolgen und überprüfen zu können, ist es von Vorteil, jedem Marker eine eigene Identifizierung im Trackingsystem zuzuordnen.
Die Soll-Eigenschaften der Marker können als Marker-Grundeigenschaften, also als Eigenschaften eines Markers ohne Verschmutzungen bzw. eines neuen Markers in einem der Datenverarbeitungseinheit zugeordneten Speicher abgelegt sein. In einem solchen Speicher können die Ist-Eigenschaften der Marker auch periodisch oder kontinuierlich gespeichert werden, wobei dann die Möglichkeit besteht, als Soll-Eigenschaften vorab gespeicherte Ist-Eigenschaften zu verwenden. Dadurch lassen sich plötzliche Veränderungen der Markereigenschaften bzw. der Markerqualität erfassen.

Wenn ein Formvergleich durchgeführt wird, kann hierzu eine typische Helligkeitsverteilung eines Markers über einen Pixelbereich des Markerabbildes herangezogen werden, und dieses Markerabbild kann durch eine (Video-)Bilderfassungseinheit des Trackingsystems oder eine separate und dem Trackingsystem zugeordnete Video-Bilderfassungseinheit erfasst werden. Bei einer Ausführungsform der vorliegenden Erfindung werden die Marker zur Bestimmung ihrer Eigenschaften in die Nähe einer Bilderfassungseinheit des Trackingsystems verbracht, um sich die höhere Genauigkeit der Abtastung in diesem Bereich zu Nutze zu machen.

Die Datenverarbeitungseinheit und/oder der ihr zugeordnete Speicher, können als separate Einheit bereitgestellt werden oder als ein Teil des Trackingsystems bzw. als ein Teil eines dem Trackingsystem zugeordneten medizinischen Navigationssystems. Es besteht aber auch die Möglichkeit, die Daten teilweise auf mehreren dieser Einrichtungen zu verarbeiten.

Gemäß einer Variante des erfindungsgemäßen Verfahrens wird eine Markerqualitäts-Abweichung festgestellt, wenn die Ist-Eigenschaften nicht den Soll-Eigenschaften entsprechen, insbesondere wenn die Abweichung bei oder über einem Schwellwert liegt. Der Schwellwert kann vorher definiert werden oder durch einen Benutzer einstellbar sein. Bei anderen Ausführungsformen wird der Schwellwert für typische Anwendungsfälle, nach Messvorgängen in der Trackingsystemumgebung und/oder nach einem Vergleich unterschiedlicher Marker in der Trackingsystemumgebung durch die Datenverarbeitungseinheit, insbesondere automatisch, eingestellt.

Wenn eine Markerqualitäts-Abweichung festgestellt wurde, können unterschiedliche Maßnahmen ergriffen werden. Diese Maßnahmen umfassen die Anzeige der Marker-Qualitätsabweichung (visuell auf einem Bildschirm; akustisch, etc.) oder die Aufforderung zum Ersatz betroffener Marker. Es kann auch (automatisch) dafür gesorgt werden, dass betroffene Marker nicht mehr vom Trackingsystem weiter verwendet werden, wenn dies möglich ist, also wenn eine entsprechende Redundanz zur Verfügung steht. Eine weitere Option besteht darin, die von den Markern definierten Positionsangaben unter Berücksichtigung der quantitativen Abweichungen rechnerisch zu korrigieren, insbesondere durch eine Neuberechnung des Abbildungszentrums. In einem Fall wird beispielsweise das Abbildungszentrum dadurch neu ermittelt, dass die noch vorhandenen, in den Ist- und Soll-Eigenschaften übereinstimmenden Teile der Markeraußenkontur benutzt werden, um das Abbildungszentrum zu ermitteln, während nicht mehr übereinstimmende Teile der Kontur dabei vernachlässigt werden. Ein solcher Vorgang ist eine, auch separat und unabhängig vom oben beschriebenen Verfahren erfinderisch definierbare, Möglichkeit zur Verbesserung der Trackinggenauigkeit bzw. zu deren Wiederherstellung.

Das verwendete Trackingsystem kann ein Bereichssensor-Trackingsystem, insbesondere ein CCD- oder CMOS-Sensorsystem, oder ein Linienscan-Trackingsystem sein. Auch was die Verwendung der speziellen Trackingsystemart betrifft, ist die vorliegende Erfindung sehr offen. Verwendet werden können optische Trackingsysteme mit passiv reflektierenden oder mit aktiv abstrahlenden Markern, magnetische Trackingsysteme, Ultraschall-, Laser- oder Radartrackingsysteme oder Kombinationen aus zweien oder mehreren dieser Trackingsysteme.

Die Erfindung betrifft ferner ein Programm, das, wenn es auf einem Computer läuft oder in einem Computer geladen ist, den Computer veranlasst, ein Verfahren durchzuführen, wie es oben in verschiedenen Ausführungsformen beschrieben worden ist. Ebenfalls umfasst sie ein Computerprogramm-Speichermedium, das ein solches Programm aufweist.

Die medizintechnische Markertrackingvorrichtung gemäß der vorliegenden Erfindung weist ein medizintechnisches Markertrackingsystem auf, das Instrumenten- oder Patientenmarker erfasst. Sie umfasst ferner eine Datenverarbeitungseinheit mit einer Logik, die Ist-Eigenschaften der Marker mit Soll-Eigenschaften vergleicht und als Ausgabe das Ergebnis des Vergleichs für die Weiterverwendung der Informationen über die erfassten Marker bereitstellt. Natürlich lassen sich alle hierin und oben verfahrensmäßig beschriebenen Merkmale der Erfindung auch vorrichtungsmäßig umsetzen.

Die Erfindung wird im Weiteren anhand der Beschreibung von Ausführungsmöglichkeiten detaillierter erläutert. Sie kann alle hierin beschriebenen Merkmale einzeln oder in jedweder sinnvollen Kombination umfassen, ebenso wie die Verwendung der beschriebenen Vorrichtungen und Verfahren für das medizintechnische Markertracking.

In den beiliegenden Zeichnungen zeigen:
- Figur 1: eine schematische Darstellung eines erfindungsgemäßen medizintechnischen Trackingsystems mit Anschluss an ein medizintechnisches Navigationssystem; und
- Figur 2: eine Gegenüberstellung von unterschiedlichen Methoden zur Ermittlung eines Abbildungszentrums (Schwerpunkt) für eine Markerabbildung.

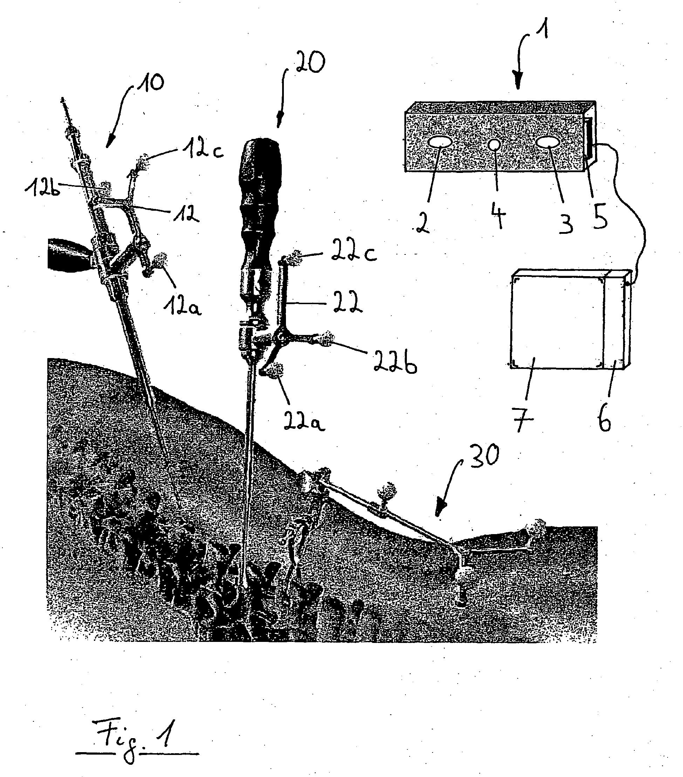

In der Figur 1 ist eine typische medizintechnische Markertracking-Umgebung dargestellt. Gezeigt sind Instrumente, die gerade bei einer navigierten und bildunterstützten Wirbelsäulenbehandlung eingesetzt werden. Beispielhaft sind zwei Instrumente 10 und 20 nummeriert worden, die jeweils eine Referenzanordnung 12 und 22 tragen. Die Referenzanordnungen 12, 22 tragen jeweils an abstehenden Armen Reflektionsmarkerkugeln, die an der Referenzanordnung 12 mit den Bezugszeichen 12a, 12b und 12c versehen worden sind. Die Referenzanordnung 22 hat drei Reflektionsmarkerkugeln 22a, 22b und 22c.

Die Marker an den Referenzanordnungen 12 und 22 sind für das jeweilige Instrument 10 bzw. 20 jeweils in eigener, charakteristischer Anordnung bereitgestellt, die es gestattet, den Anordnungen jeweils ein spezielles Instrument, hier das Instrument 10 bzw. 20, eindeutig zuzuordnen.

Schematisch sind in Figur 1 noch ein Trackingsystem 1 und ein Navigationssystem 6 mit Bildschirmausgabe 7 dargestellt. Das Trackingsystem besteht aus einem Gehäuse mit zwei Kameras 2, 3 und einem Infrarotlichterzeuger (Blitz) 4. Der Infrarotlichterzeuger 4 erzeugt in sehr kurzen Abschnitten Infrarotlichtblitze, die von den reflektierenden Markern an den Referenzanordnungen 12, 22 reflektiert werden. Diese Reflektionen werden durch die stereoskopischen Kameras 2, 3 erfasst, und das Trackingsystem 1 berechnet (nach einer Kalibrierung) die räumlichen Koordinaten der Marker für die Instrumente 10, 20. Diese errechneten Positionsdaten werden an das Navigationssystem 6 weitergegeben, welches den Markeranordnungen 12, 22 die Instrumente 10, 20 zuordnet und deren Position im Verhältnis zu der Lage der Patientenanatomie auf dem Bildschirm 7 wiedergibt. Die Lage der interessierenden Patientenanatomie (hier der Wirbelsäule) wird beispielsweise durch eine Referenzanordnung ermittelt, die in Figur 1 mit dem Bezugszeichen 30 versehen worden ist.

Im Trackingsystem ist eine Datenverarbeitungseinheit untergebracht, welche schematisch dargestellt und mit dem Bezugszeichen 5 versehen worden ist. Diese Datenverarbeitungseinheit weist auch einen Datenspeicher auf. In dem Speicher der Datenverarbeitungseinheit 5 sind erfindungsgemäß Daten über die Soll-Eigenschaften von Markern hinterlegt. Im vorliegenden Fall wären dies Daten darüber, wie mehrere oder einer der Marker 12a, b, c bzw. 22a, b, c durch das Trackingsystem abgebildet werden sollten. Es ist an dieser Stelle anzumerken, dass die Daten nicht unbedingt im Trackingsystem selbst bzw. in einer dem Trackingsystem zugeordneten Datenverarbeitungseinheit 5 hinterlegt werden müssen. Die Datenverarbeitungseinheit kann auch separat bereitgestellt werden oder die Datenverarbeitungseinheit des Navigationssystems 6 sein. Die Kameras 2, 3 des Trackingsystems 1 können sowohl Positionsdaten als Punktkoordinaten ermitteln als auch über gewisse Pixelbereiche Helligkeitswerte und Formen wahrnehmen. Außerdem ist die Ermittlung der Helligkeit, der Größe und der Sichtbarkeit (sichtbar oder nicht sichtbar) möglich, und aus geeignet kombinierten Informationen kann auch der Verschmutzungsgrad ermittelt werden.

Wenn diese Eigenschaften oder eine Kombination dieser Eigenschaften (Ist-Eigenschaften) nicht den vorgegebenen Soll-Eigenschaften entsprechen oder die Abweichungen einen Schwellwert überschreiten, können geeignete Maßnahmen getroffen werden, beispielsweise kann die Abweichung oder der Trackingfehler auf dem Bildschirm 7 des Navigationssystems ausgegeben werden. Andere Möglichkeiten sind Aufforderungen zum Ersatz des betreffenden Markers oder die Herausnahme des Markers aus der Berechnung, wenn es möglich ist, mit den restlichen Markern noch korrekt zu navigieren.

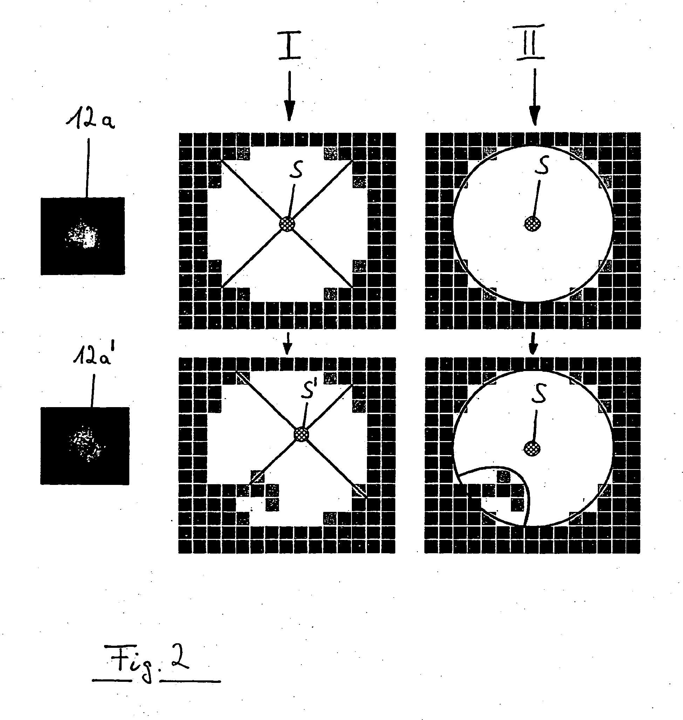

Eine spezielle Möglichkeit, die sich bei der Feststellung einer Marker-Qualitätsabweichung anbietet, ist die Neubestimmung des Abbildungszentrums, das im Weiteren auch als Flächenschwerpunkt oder Schwerpunkt bezeichnet wird. Die Kameras des Trackingsystems 1 erfassen ein Bild, das sich über mehrere Pixel erstreckt. In der Figur 2 ist in der oberen Reihe links ein Marker 12a gezeigt, wie er im Grundzustand, also als neuer oder unverschmutzter Marker auszusehen hat. In den beiden vergrößerten Darstellungen rechts daneben ist zu sehen, dass der Marker bei seiner Abbildung, also im Kamerabild eine Anzahl von Pixeln mit einer gewissen Helligkeit belegt, und in der mit I bezeichneten Spalte wird oben dargestellt, wie der Schwerpunkt (Flächenschwerpunkt) herkömmlicherweise bestimmt wird. Hierzu werden zwei sich orthogonal schneidende Geraden in den Bereich der hellen Pixel eingebracht, und am Schnittpunkt wird die Lage des Schwerpunktes S und damit die Lage des Abbildungszentrums definiert. Diesem Punkt werden dann die entsprechenden Marker-Positionskoordinaten zugeordnet.

In der unteren Reihe in Figur 2 ist nun ein Zustand gezeigt, der für einen verschmutzten Marker gilt. Der Marker 12a' hat am unteren linken Ende eine Verschmutzung (beispielsweise durch einen Blutfleck), was die Helligkeit in diesem Bereich absenkt. Wenn man nun in derselben Weise wie oben die beiden sich kreuzenden Geraden einzeichnet und den Schnittpunkt bildet, ergibt sich ein Flächenschwerpunkt S', der aus dem tatsächlichen Zentrum des Markers 12a heraus verschoben ist. Die sich hieraus ergebenden Koordinaten für den Schwerpunkt S' sind fehlerhaft und können zu Navigations- bzw. Trackingmängeln führen.

In der Spalte, die mit II bezeichnet ist, wird nun eine erfindungsgemäße Lösung aufgezeigt. Bei dieser Lösung wird für einen Marker 12a (nicht verschmutzt) die äußere Kontur ermittelt, und im vorliegenden Fall (nicht verschmutzter Marker, oberes rechtes Bild ergibt sich aus der Helligkeitsverteilung eine Kreiskontur. Bei einer solchermaßen definierten Kontur, die computergestützt aus den Pixel-Helligkeiten ermittelt werden kann, lässt sich auch ein eindeutiges Zentrum ermitteln, und dieses Zentrum ist im vorliegenden Fall der Kreismittelpunkt, der wiederum als Flächenschwerpunkt S aufgezeigt ist.

Beim verschmutzten Marker sind zwar im linken unteren Bereich uneinheitliche und unregelmäßige Helligkeitsverteilungen vorhanden, jedoch lässt sich die Außenkontur, also die Kreiskontur für den restlichen Bereich noch sehr gut ermitteln bzw. zuordnen. Erfindungsgemäß wird nun dieser Bereich, der eine eindeutig zuordnungsfähige Kontur ermittelbar macht, wiederum verwendet, um den Schwerpunkt S zu bestimmen; der restliche Bereich wird aus dieser Ermittlung bzw. Berechnung ausgegrenzt. Hierbei ist es von besonderem Vorteil, dass im Trackingsystem auch Daten darüber hinterlegt sind, wie die Markerabbildung eigentlich auszusehen hat (Soll-Eigenschaft), so dass sich der für die Bestimmung des Schwerpunktes zu verwendende, verbleibende Konturabschnitt auch mit Unterstützung dieser Informationen ermitteln lässt. Wie im unteren Bereich der Spalte II zu sehen ist, wird der Schwerpunkt bei einer solchen rechnerischen Korrektur bzw. Bestimmung wieder an die richtige Stelle gesetzt und das Tracking bzw. die Navigation können weiterhin mit korrekten Positionsdaten durchgeführt werden.

Die vorliegende Erfindung und ihre Ausführungsformen machen es möglich, Marker eines medizintechnischen Trackingsystems basierend auf verschiedenen Indikatoren qualitativ einzuordnen, die beispielsweise aus typischen Setups für Trackingsysteme ermittelt werden oder auch aus Erfahrungen mit speziellen Trackingsystemen. Sie stellt mit anderen Worten ein Expertensystem zur Verfügung, das die folgenden Informationen und Überlegungen berücksichtigt:
1. Die Helligkeit von Markern (beispielsweise von Reflektionsmarkern) sollte im Trackingvolumen des Trackingsystems innerhalb gewisser Grenzen liegen. Da die Raumposition der Marker durch das Trackingsystem ermittelt wird, kann die zu erwartende Helligkeit eines vorgegebenen Markers mit seiner tatsächlichen Helligkeit verglichen werden. Die Helligkeit eines Markers hängt von seiner Form (Scheibe, Kugel, Würfel, Pyramide, ....), seiner Größe und von den Reflektionseigenschaften bzw. Abstrahleigenschaften seiner Oberfläche ab. Jedoch wird bei den meisten Anwendungen nur ein Markertyp verwendet, oder die Markertypen sind aus bekannten oder hinterlegten Starrkörperdatensätzen für unterschiedliche Instrumente mit unterschiedlichen Markern bekannt. Deshalb kann ein Expertensystem die zu erwartende Helligkeit eines vorgegebenen Markers bei einer vorgegebenen Position im Raum mit der tatsächlichen Helligkeit vergleichen, die vom Trackingsystem ermittelt wird.
2. Das System kann zusätzlich Videobilder verwenden, die von den Trackingsystemsensoren erfasst werden, um eine typische Helligkeitsverteilung eines Markers über einen Pixelsatz zu bestimmen. Wenn bekannt ist, dass der Marker rund (Kugel) oder elliptisch (Scheibenmarker) ist und die Auflösung des Sensors bzw. Subpixelallgorithmen, die auf den Sensordaten ausgeführt werden, es gestatten, unerwartete Formen zu entdecken, kann diese Information verwendet werden, um ein weiteres Qualitätskriterium (Eigenschaft) des Markers zu definieren.
3. Aufgrund von technischen Einschränkungen ist die Auflösung insbesondere älterer Trackingsysteme relativ gering und Algorithmen für die Kantenerfassung und anderen Formerkennungen funktionieren speziell dann nicht besonders gut, wenn die Marker relativ weit von den Sensoren weg sind. In solchen Fällen ist es möglich, die Markerqualität für Instrumente zu bestimmen, indem man die Marker nahe an die Kamera des Trackingsystems heranbringt. In diesem Modus (close-up-Mode) können die exakten Abstände zwischen den Markern auf ihrem Starrkörper bestimmt und jedwede Ungenauigkeiten während der Bewegung errechnet werden. Damit erhält man einen guten Indikator dafür, ob potentielle Probleme mit verschmutzten Markern auftreten können, und zwar sogar mit einer relativ geringen Sensorauflösung.
4. Ein typisches Problem liegt beim Markertracking darin, dass ein Marker plötzlich während der Behandlung verschmutzt oder nass wird. Durch die vorliegende Erfindung können solche plötzlichen Änderungen der optischen Eigenschaften (Helligkeit, Farbe, Form) durch das Trackingsystem erkannt werden. Das Trackingsystem speichert dazu die über den Marker gesammelten Informationen periodisch oder kontinuierlich über die Verwendungsdauer und kann die jeweils neu erfassten Eigenschaften mit vorher erfassten Daten vergleichen. Dabei ist es wichtig, dass das Trackingsystem identifiziert, welcher Marker eines Datensatzes dem Marker im anderen Datensatz entspricht. Deshalb werden bevorzugt alle Marker eindeutig identifiziert. Das System bezieht sich hierbei auf die jeweils eindeutigen und nur einmal vorkommenden Abstände zwischen Markern an den Referenzanordnungs-Starrkörpern und hat die Möglichkeit, eine Nummerierung eines Markers während der Verwendung sogar dann aufrecht zu erhalten, wenn die Instrumente oder das Trackingsystem bewegt werden. Wie in der Figur 1 dargestellt, erhalten die Marker einzelne Bezeichnungen (12a, 12b, 12c, 22a, 22b und 22c).
   Für den Arbeitsablauf ist es wichtig, die Marker, die momentan verwendet werden zu identifizieren und ihre Nummern aufrecht zu erhalten, und zwar sogar dann, wenn sie zeitweise nicht sichtbar sind. Wenn beispielsweise das Instrument 10 für eine Weile wegen einer Abdeckung oder weil es gerade nicht verwendet wird, nicht sichtbar ist, bleibt doch die Nummerierung seiner Marker wirksam, wenn es wieder in das Kamera-Sichtfeld eintritt. Deshalb können Änderungen der optischen Eigenschaften der Instrumentenmarker von einem Datensatz zum Nächsten wiederum identifiziert werden, und dies sogar über längere Zeitabstände, wenn das Instrument für eine Weile nicht verwendet worden ist, z.B. wenn das Instrument gereinigt wurde, während ein Chirurg mit einem anderen Instrument gearbeitet hat.
5. Wenn Sensoren mit hoher Auflösung verwendet werden und gute Sub-Pixel-Bildverarbeitungsmechanismen zur Verfügung stehen, können die herkömmlichen Schwerpunkts-Ermittlungs-Allgorithmen zur Abschätzung der Markerposition merklich verbessert werden. Kombiniert mit diesen neuen Technologien aus anderen technischen Gebieten, kann dabei auch die Erfassung von Verschmutzungen auf Markern weiter verbessert werden.
   Basierend auf den obigen Überlegungen und technischen Ausführungen kann ein vollständiger Satz von Qualitätskriterien für jeden Marker definiert werden, und das Trackingsystem kann die Kriterien kontinuierlich vergleichen und gegebenenfalls Warnungen oder andere Weisungen/Informationen für den Benutzer ausgeben.
   Zusammenfassend lässt sich sagen, dass die Erfindung es dem Verwender gestattet, Probleme mit Markern zu erkennen, die bei bisherigen Markertrackingsystemen nicht offensichtlich waren. Es wird möglich, plötzliche Änderungen der optischen Markereigenschaften aufgrund von Verschmutzungen der Marker zu erfassen, was insbesondere für solche Fälle gilt, wo die Marker durch Wassertropfen benetzt werden, was ansonsten für die Verwender nicht leicht erkennbar war. Es wird möglich, potentielle Marker-Okklusionen zu erfassen und dem Verwender Informationen über die Qualität hinsichtlich der Trackinggenauigkeit zur Verfügung zu stellen. Dadurch erhöht sich auch die Sicherheit der Tracking- und Navigationssysteme, und zwar aufgrund der erhöhten Genauigkeit und der Vermeidung von falschen Positionsermittlungen. Ebenfalls wird die Zuverlässigkeit von Tracking- und Navigationssystem verbessert, weil Informationen über die Qualität des Instrumententrackings zur Verfügung stehen.

## Patentansprüche

1. Medizintechnisches Markertrackingverfahren bei dem mittels eines medizintechnischen Markertrackingsystems (1) Instrumenten- oder Patientenmarker (12a,b,c; 22a,b,c) erfasst werden, wobei mittels einer Datenverarbeitungseinheit (5) die Ist-Eigenschaften der Marker (12a,b,c; 22a,b,c) mit Soll-Eigenschaften verglichen werden und das Ergebnis des Vergleichs der Weiterverwendung der Informationen über die erfassten Marker (12ab,c; 22a,b,c) zugrunde gelegt wird, wobei eine Markerqualitäts-Abweichung festgestellt wird, wenn die Ist-Eigenschaften nicht den Soll-Eigenschaften entsprechen, insbesondere wenn die Abweichung über einem Schwellwert liegt, wobei nach der Feststellung einer Markerqualitäts-Abweichung die von den Markern definierten Positionsangaben unter Berücksichtigung der quantitativen Abweichungen rechnerisch korrigiert werden, durch eine Neuberechnung des Abbildungszentrums.

2. Verfahren nach Anspruch 1, bei dem die zu vergleichenden Eigenschaften umfassen:
- die Helligkeit bzw. den Helligkeitsverlauf in den sensorisch erfassten Markermerkmalen,
- die Form,
- die Größe
- die Sichtbarkeit,
- den Verschmutzungsgrad,
- die Korrelation von Abbildungen eines Markers aus verschiedenen Blickrichtungen bzw. die Sehstrahlabweichung oder Epipolargeometrie; und
- die Position bzw. relative Position zu einer Referenz
eines Markers bzw. dessen Abbildung im Trackingsystem, oder eine Kombination aus zweien oder mehreren dieser Eigenschaften.

3. Verfahren nach Anspruch 1 oder 2, bei dem jedem Marker eine eigene Identifizierung zugeordnet wird.

4. Verfahren nach einem der Ansprüche 1 bis 3, bei dem die Soll-Eigenschaften der Marker (12a,b,c; 22a,b,c) als Marker-Grundeigenschafien in einem der Datenverarbeitungseinheit (5) zugeordneten Speicher abgelegt sind.

5. Verfahren nach einem der Ansprüche 1 bis 4, bei dem die Ist-Eigenschaften der Marker periodisch oder kontinuierlich in einem der Datenverarbeitungseinheit (5) zugeordneten Speicher gespeichert werden.

6. Verfahren nach Anspruch 5, bei dem als Soll-Eigenschaften zuvor abgespeicherte Ist, Eigenschaften verwendet werden, um eine plötzliche Veränderung der Markereigenschaften zu erfassen.

7. Verfahren nach einem der Anspruche 2 bis 6, bei dem zum Formvergleich eine typische Helligkeitsverteilung eines Markers (12a,b,c; 22a,b,c) über einen Pixelbereich des Markerabbilds herangezogen wird, das durch eine Video-Bilderfassungseinheit des Trackingsystems (1) oder eine separate Video-Bilderfassungseinheit erfasst wird.

8. Verfahren nach einem der Ansprüche 1 bis 7, bei dem die Marker (12a,b,c; 22a,b,c) zur Bestimmung ihrer Eigenschaften in die Nähe einer Bilderfassungseinheit des Trackirigsystems gebracht werden.

9. Verfahren nach einem der Ansprüche 1 bis 8, bei dem die Datenverarbeitungseinheit (5) und/oder der ihr zugeordnete Speicher als separate Einheit, als Teil des Trackingsystems (1) oder als Teil eines dem Trackingsystem (1) zugeordneten medizintechnischen Navigationssystems (6) bereitgestellt wird, oder die Daten teilweise auf mehreren dieser Einrichtungen verarbeitet werden.

10. Verfahren nach Anspruch 1, bei dem der Schwellwert vordefiniert ist oder durch einen Benutzer einstellbar ist.

11. Verfahren nach Anspruch 1 oder 11, bei dem der Schwellwert für typische Anwendungsfälle, nach Messvorgängen in der Trackingsystemumgebung und/oder nach einem Vergleich unterschiedlicher Marker in der Trackingsystemumgebung durch die Datenverarbeitungseinheit, insbesondere automatisch, eingestellt wird.

12. Verfahren nach einem der Ansprüche 1 bis 11, bei dem nach der Feststellung einer Markerqualitäts-Abweichung einer oder mehrere der folgenden Schritte durchgeführt werden:
- die Marker-Qualitätsabweichung wird angezeigt,
- betroffene Marker werden, wenn möglich, vom Trackingsystem nicht weitere verwendet,
- es wird zum Ersatz betroffener Marker aufgefordert.

13. Verfahren nach Anspruch 1, bei dem das Abbildungszentrum dadurch neu ermittelt wird, dass die noch vorhandenen, in den Ist- und Soll-Eigenschaften übereinstimmenden Teile der Markeraußenkontur benutzt werden, um das Abbildungszentrum zur ermitteln, während nicht mehr übereinstimmende Teile der Kontur dabei vernachlässigt werden.

14. Verfahren nach einem der Ansprüche 1 bis 13, bei dem das Trackingsystem ein Bereichssensor-Trackingsystem, insbesondere ein CCD- oder CMOS-Sensorsystem, oder ein Linienscan-Trackingsystem ist.

15. Verfahren nach einem der Ansprüche 1 bis 14, bei dem das Trackingsystem ein
- optisches Trackingsystem mit passiv reflektierenden Markern,
- optisches Trackingsystem mit aktiv abstrahlenden Markern,
- magnetisches Trackingsystem;
- Ultraschalltrackingsystem,
- Lasertrackingsystem oder ein
- Radartrackingsystem
ist oder eine Kombination aus diesen Trackingsystemen umfasst.

16. Programm, das, wenn es auf einem Computer läuft oder in einem Computer geladen ist, den Computer veranlasst, ein Verfahren gemäß einem der Ansprüche 1 bis 15 durchzuführen.

17. Computerprogramm-Speichermedium, das ein Programm nach Anspruch 16 aufweist.

18. Medizintechnische Markertrackingvorrichtung mit einem medizintechnischen Markertrackingsystem (1), das Instrumenten- oder Patientenmarker (12a,b,c; 22a,b,c) erfasst, , und einem Navigationssystem (6) mit Bildschirm (7), wobei das Markertrackingsystem (1) eine Datenverarbeitungseinheit (5) mit einer Logik aufweist, die konfiguriert ist, Ist-Eigenschaften der Marker (12a,b,c; 22a,b,c) mit Soll-Eigenschaften zu vergleichen und als Ausgabe das Ergebnis des Vergleichs für die Weiterverwendung der Informationen über die erfassten Marker (12a,b,c; 22a,b,c) bereitzustellen, wobei eine Markerqualitäts-Abweichung feststellbar ist, wenn die Ist-Eigenschaften nicht den Soll-Eigenschaften entsprechen, insbesondere wenn die Abweichung über einem Schwellwert liegt, wobei nach der Feststellung einer Marker-Qualitätsabweichung die Datenverarbeitungseinheit (5) konfiguriert ist, die von den Markern definierten Positionsangaben unter Berücksichtigung der quantitativen Abweichungen rechnerisch zu korrigieren, durch eine Neuberechnung des Abbildungszentrums.

## Claims

1. A medical marker tracking method, in which instrument or patient markers (12a,b,c; 22a,b,c) are detected by means of a medical marker tracking system (1), wherein the actual properties of the markers (12a,b,c; 22a,b,c) are compared with nominal properties by means of a data processing unit (5), and the result of the comparison is used as a basis for the subsequent use of the information concerning the detected markers (12a,b,c; 22a,b,c), wherein a deviation in marker quality is determined if the actual properties do not correspond to the nominal properties, in particular if the deviation is above a threshold value, wherein once a deviation in marker quality has been determined, the positional details defined by the markers are arithmetically corrected, taking into account the quantitative deviations, by recalculating the image centre.

2. The method according to claim 1, wherein the properties to be compared include:
- the brightness and/or brightness profile in the marker features detected by sensors;
- the shape;
- the size;
- the visibility;
- the degree of soiling;
- the correlation of images of a marker from various viewing directions and/or the line-of sight deviation or epipolar geometry; and
- the position and/or position relative to a reference
of a marker and/or its image in the tracking system, or a combination of two or more of these properties.

3. The method according to claim 1 or 2, wherein each marker is assigned a unique identifier.

4. The method according to any one of claims 1 to 3, wherein the nominal properties of the markers (12a,b,c; 22a,b,c) are stored as base properties of the markers in a memory which is assigned to the data processing unit (5).

5. The method according to any one of claims 1 to 4, wherein the actual properties of the markers are periodically or continuously stored in a memory which is assigned to the data processing unit (5).

6. The method according to claim 5, wherein previously stored actual properties are used as nominal properties, in order to detect a sudden change in the marker properties.

7. The method according to any one of claims 2 to 6, wherein a typical brightness distribution of a marker (12a,b,c; 22a,b,c) over a pixel range of the marker image, which is detected by a video image detection unit of the tracking system (1) or by a separate video image detection unit, is adduced in order to compare the shape.

8. The method according to any one of claims 1 to 7, wherein the markers (12a,b,c; 22a,b,c) are moved into the vicinity of an image detection unit of the tracking system, in order to determine their properties.

9. The method according to any one of claims 1 to 8, wherein the data processing unit (5) and/or the memory assigned to it is provided as a separate unit, as a part of the tracking system (1) or as a part of a medical navigation system (6) which is assigned to the tracking system (1), or the data are partially processed on a number of these devices.

10. The method according to claim 1, wherein the threshold value is pre-defined or can be set by a user.

11. The method according to claim 1 or 11, wherein the threshold value for typical applications is set by the data processing unit, in particular automatically, after measuring procedures in the tracking system environment and/or after comparing different markers in the tracking system environment.

12. The method according to any one of claims 1 to 11, wherein once a deviation in marker quality has been determined, one or more of the following steps are performed:
- the deviation in marker quality is indicated;
- affected markers are, if possible, no longer used by the tracking system;
- the replacement of affected markers is requested.

13. The method according to claim 1, wherein the image centre is recalculated by using the parts of the marker's outer contour which are still available and match in terms of the actual and nominal properties to calculate the image centre, while disregarding parts of the contour which no longer match.

14. The method according to any one of claims 1 to 13, wherein the tracking system is a region sensor tracking system, in particular a CCD or CMOS sensor system, or a line scanning tracking system.

15. The method according to any one of claims 1 to 14, wherein the tracking system is:
- an optical tracking system comprising passively reflecting markers;
- an optical tracking system comprising actively emitting markers;
- a magnetic tracking system;
- an ultrasound tracking system;
- a laser tracking system; or
- a radar tracking system;
or includes a combination of these tracking systems.

16. A program which, when it is running on a computer or is loaded onto a computer, causes the computer to perform a method in accordance with any one of claims 1 to 15.

17. A computer program storage medium, comprising a program according to claim 16.

18. A medical marker tracking device, comprising a medical marker tracking system (1) which detects instrument or patient markers (12a,b,c; 22a,b,c), and a navigation system (6) comprising a screen (7), wherein the medical marker tracking system (1) comprises a data processing unit (5) which includes a logic which is configured to compare actual properties of the markers (12a,b,c; 22a,b,c) with nominal properties and to provide the result of the comparison as an output for the subsequent use of the information concerning the detected markers (12a,b,c; 22a,b,c), wherein a deviation in marker quality may be determined if the actual properties do not correspond to the nominal properties, in particular if the deviation is above a threshold value, wherein once a deviation in marker quality has been determined, the data processing unit (5) is configured to arithmetically correct the positional details defined by the markers, taking into account the quantitative deviations, by recalculating the image centre.

## Revendications

1. Procédé médical de suivi de repères dans lequel on détecte des repères d'instrument ou de patient (12a, b, c ; 22a, b, c) au moyen d'un système médical de suivi de repères (1), on compare, au moyen d'une unité de traitement de données (5), des propriétés réelles des repères (12a, b, c ; 22a, b, c) à des propriétés de consigne, et on prend le résultat de la comparaison pour base de l'utilisation complémentaire des informations relatives aux repères détectés (12a, b, c ; 22a, b, c), dans lequel on constate un écart de qualité des marqueurs lorsque les propriétés réelles ne correspondent pas aux propriétés de consigne, en particulier lorsque l'écart se situe au-dessus d'une valeur de seuil, dans lequel après constatation d'un écart de qualité des repères, on corrige par le calcul les indications de position définies par les repères en tenant compte des écarts quantitatifs, par un nouveau calcul du centre de l'image.

2. Procédé selon la revendication 1, dans lequel les propriétés à comparer comprennent :
- la luminosité ou la variation de la luminosité dans les caractéristiques des repères repérées par capteur,
- la forme,
- la taille,
- la visibilité,
- le degré d'encrassement,
- la corrélation des images d'un repère à partir de différentes directions d'observation ou la géométrie épipolaire ; et
- la position ou position relative par rapport à une référence
d'un repère ou de son image dans le système de suivi, ou une combinaison de deux ou plus de ces propriétés.

3. Procédé selon l'une quelconque des revendications 1 ou 2, dans lequel une identification propre est associée à chaque repère.

4. Procédé selon l'une quelconque des revendications 1 à 3, dans lequel les propriétés de consigne des repères (12a, b, c ; 22a, b, c) sont mémorisées en tant que propriétés de base des repères dans une mémoire associée à l'unité de traitement de données (5).

5. Procédé selon l'une quelconque des revendications 1 à 4, dans lequel les propriétés réelles des repères sont mémorisées périodiquement ou en continu dans une mémoire associée à l'unité de traitement de données (5).

6. Procédé selon la revendication 5, dans lequel on utilise, en tant que propriétés de consigne, des propriétés réelles préalablement mémorisées, afin de détecter une variation brusque des propriétés des repères.

7. Procédé selon l'une quelconque des revendications 2 à 6, dans lequel, pour la comparaison des formes, on a recours à une distribution caractéristique de la luminosité d'un repère (12a, b, c ; 22a, b, c) sur une zone de pixels de l'image des repères, qui est détectée par une unité vidéo de prise de vue du système de suivi (1) ou une unité vidéo de prise de vue séparée.

8. Procédé selon l'une quelconque des revendications 1 à 7, dans lequel les repères (12a, b, c ; 22a, b, c) sont amenés, pour déterminer leurs propriétés, à proximité d'une unité de prise de vue du système de suivi.

9. Procédé selon l'une quelconque des revendications 1 à 8, dans lequel l'unité de traitement de données (5) et/ou la mémoire qui lui est associée en tant qu'unité séparée, est préparée en tant que partie du système de suivi (1) ou en tant que partie d'un système médical de navigation (6) associé au système de suivi (1), ou les données sont traitées partiellement sur plusieurs de ces dispositifs.

10. Procédé selon la revendication 1, dans lequel la valeur de seuil est prédéfinie ou peut être réglée par un utilisateur.

11. Procédé selon l'une quelconque des revendications 1 ou 11, dans lequel la valeur de seuil pour des cas d'application caractéristiques, est réglée, en particulier automatiquement, après des opérations de mesure dans l'environnement du système de suivi et/ou après une comparaison de différents repères dans l'environnement du système de suivi, par l'unité de traitement de données.

12. Procédé selon l'une quelconque des revendications 1 à 11, dans lequel, après constatation d'un écart de qualité des repères, on exécute une ou plusieurs des étapes suivantes :
- l'écart de qualité des repères est affiché,
- si possible, les repères concernés ne sont plus utilisés par le système de suivi,
- le remplacement des repères concernés est demandé.

13. Procédé selon la revendication 1, dans lequel on détermine à nouveau le centre de l'image en ce qu'on utilise les parties du contour extérieur des repères qui coïncident dans les propriétés réelles et les propriétés de consigne et qui sont encore présentes, afin de déterminer le centre de l'image, tandis qu'on néglige ici les parties non coïncidentes du contour.

14. Procédé selon l'une quelconque des revendications 1 à 13, dans lequel le système de suivi est un système de suivi à détection de zone, en particulier un système de détection CCD ou CMOS, ou un système de suivi par scanner de lignes.

15. Procédé selon l'une quelconque des revendications 1 à 14, dans lequel le système de suivi est un
- système de suivi optique avec repères réfléchissants passifs,
- système de suivi optique avec repères réfléchissants actifs,
- système de suivi magnétique,
- système de suivi à ultrasons,
- système de suivi à laser ou un
- système de suivi radar
ou comprend une combinaison de ces systèmes de suivi.

16. Programme qui lorsqu'il tourne sur un ordinateur ou est chargé dans un ordinateur fait que l'ordinateur exécute un procédé selon l'une quelconque des revendications 1 à 15.

17. Support de mémoire de programme d'ordinateur qui comporte un programme selon la revendication 16.

18. Dispositif médical de suivi de repères avec un système médical de suivi de repères (1) qui détecte des repères d'instrument ou de patient (12a, b, c ; 22a, b, c), et avec un système de navigation (6) à écran (7), dans lequel le système de suivi de repères (1) comporte une unité de traitement de données (5) avec une logique qui est configurée pour comparer des propriétés réelles des repères (12a, b, c ; 22a, b, c) à des propriétés de consigne et pour préparer en tant que sortie le résultat de la comparaison pour l'utilisation complémentaire des informations relatives aux repères détectés (12a, b, c ; 22a, b, c), dans lequel un écart de qualité des repères peut être constaté lorsque les propriétés réelles ne correspondent pas aux propriétés de consigne, en particulier lorsque l'écart se situe au-dessus d'une valeur de seuil, dans lequel, après constatation d'un écart de qualité des repères, l'unité de traitement de données (5) est configurée pour corriger par le calcul les indications de position définies par les repères, en tenant compte des écarts quantitatifs, par un nouveau calcul du centre de l'image.
